# EUROPEAN PATENT SPECIFICATION

(11) **EP 0 780 105 B1**
(45) Date of publication and mention of the grant of the patent: **18.09.2002**
(21) Application number: 96500163.9
(22) Date of filing: 18.12.1996
(51) Int. Cl.: A61F 2/00

(54) **Device for women suffering from incontinence**
Vorrichtung für an Inkontinenz leidende Frauen
Dispositif pour femmes souffrant d'incontinence

(30) Priority: 19.12.1995 ES 9503200 U
(43) Date of publication of application: 25.06.1997
(73) Proprietor: Doladé Guardia, José Manuel, 08017 Barcelona (ES)
(72) Inventor: Doladé Guardia, José Manuel, 08017 Barcelona (ES)
(74) Representative: Durán Olivella, Alfonso

(56) References cited:
- WO-A-92/06731
- WO-A-92/19192
- WO-A-93/24075
- US-A- 4 634 421
- US-A- 5 513 659

## Description

The present invention relates to a device for women suffering from incontinence.

The aim of the device forming the subject-matter of the present invention is, in cases of feminine incontinence, to provide the patient with a simple and practical means of avoiding the effects of said incontinence, enabling the flow of urine to be controlled by the user as desired.

WO 93/24075 discloses a urinary drainage cathether which has a flat disc-like closing end which flexes in the sides to allow introduction into the urethra.

WO 92/19192 discloses a device for preventing involuntary urination having an oblong body with diameter increasing enlargements and a disc-like closing portion, the device needing full extraction to release the contents of the bladder.

The device forming the subject-matter of the present invention is basically formed by a cylindrical sleeve, the top of which is provided with a closure head for the neck of the bladder, which head has openings communicating with the inside of the sleeve which is inserted from the outside into the patient's body until it reaches a stop position, and which has a small closure plug, preferably connected to the sleeve by a laminar extension, which enables the device to be kept closed, as desired by the patient, until, by opening the lower plug, it is possible to control the emission of urine without having to withdraw the device once it has been positioned.

In order to introduce the device, a small rod is provided which can be introduced into the sleeve and which is sufficiently rigid and strong to be able, with the aid of a small lower grip, to be used manually for the introduction of the device for controlling incontinence.

The device is preferably produced from a silicone rubber material or the like which, apart from its known features of a low coefficient of friction, can additionally be sterilised and cleaned and also recycled.

In order to clarify the invention, some explanatory drawings of the present invention are appended by way of example.

Figures 1, 2 and 3 are side, front and plan views, respectively, of the device forming the subject-matter of the present invention.

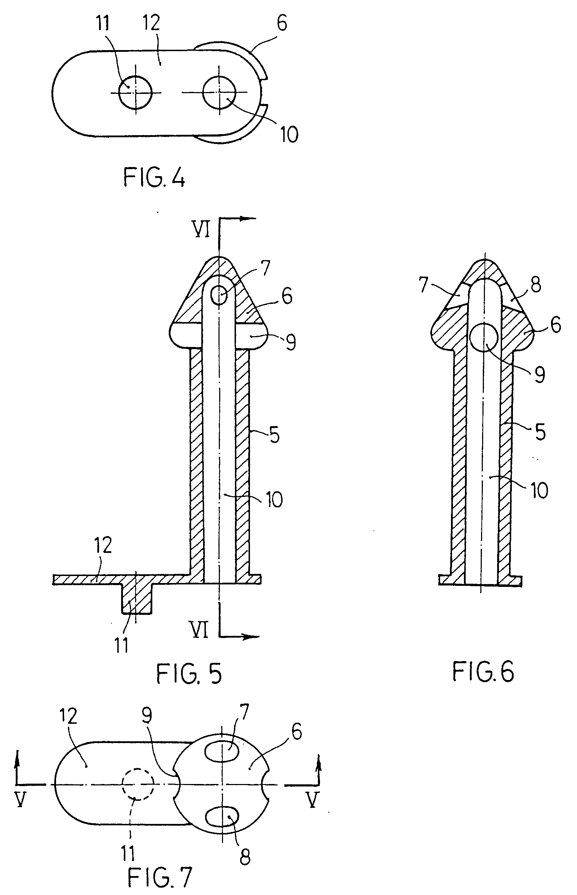

Figures 4, 5, 6 and 7 are, respectively, a bottom view, two longitudinal sections and a top view of an embodiment of the device for feminine incontinence forming the subject-matter of the present invention.

Figure 8 is a longitudinal section through the incontinence device with its internal applicator rod.

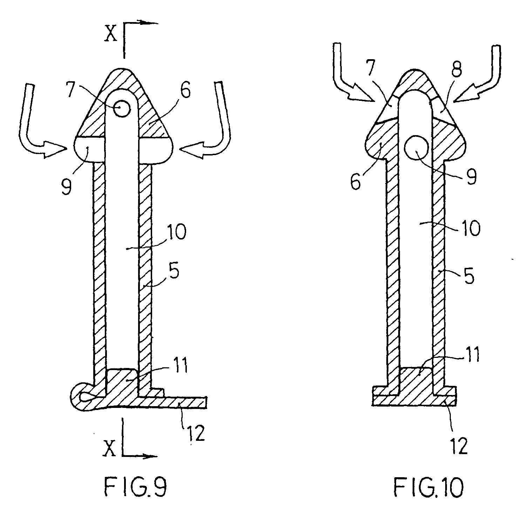

Figures 9 and 10 are each sections through the feminine incontinence device in the closed position.

Figure 11 shows diagrammatically the application of the feminine incontinence device forming the subject-matter of the present invention.

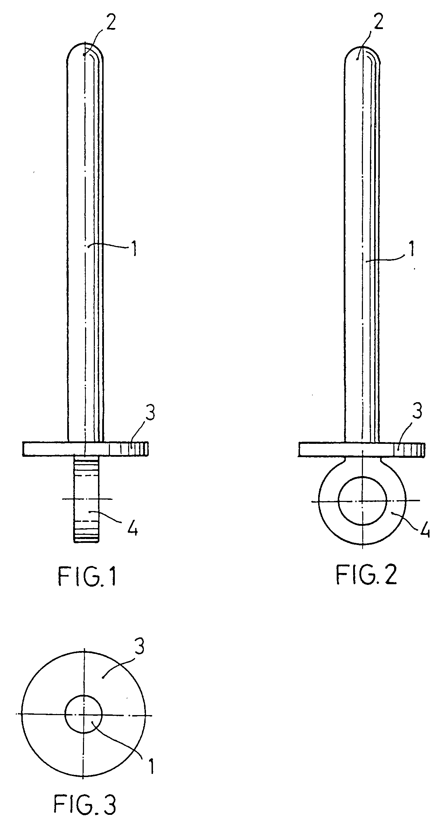

Figures 1 to 3 show the applicator element of the incontinence device, comprising a preferably cylindrical rod 1 produced from a material having a certain rigidity, the end 2 of which is rounded and which is provided with a stop flange 3 near its lower end and with a small grip 4 which may be in the form of a holding and/or recovery ring.

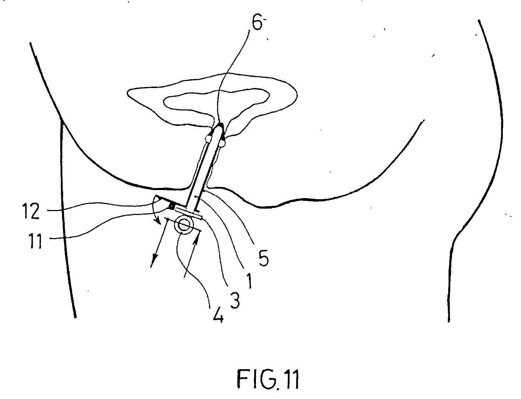

The closure element of the incontinence device comprises a cylindrical sleeve 5 provided, at its upper end, with a closure head 6 which is substantially frustoconical and which is rounded both at the base and at the apex and is provided with various transverse communication openings, such as 7, 8 and 9, preferably at two levels, which communicate with the axial hole 10 of the sleeve 5. The mentioned closure element can be introduced into the position for plugging the neck of the bladder, as shown in Figure 11, the plugging of the axial hole 10 being effected by means of a lower plugging means which may be formed by a projection 11 of an appendage 12 which is an extension of the lower portion of the actual casing 5, thus enabling plugging to be achieved without requiring elements which are separate and therefore easily mislaid.

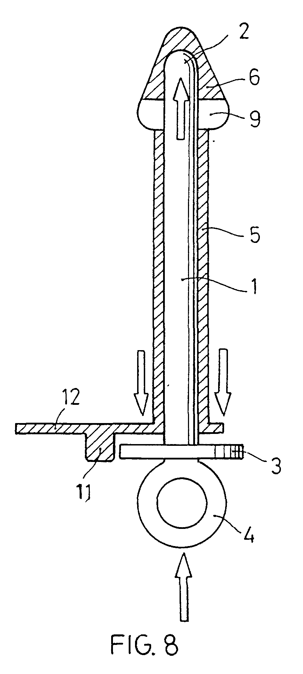

The positioning of the incontinence device is effected, as shown in Figure 8, by manipulating the internal rod 1 accommodated in the hole 10 of the sleeve 5. The mentioned introduction takes place until a stop position on the user's body is reached, at which moment the axial hole of the sleeve 5 is closed so that the device can correctly carry out its function of controlling incontinence.

As and when desired by the user, the flow of urine can be permitted by unplugging the axial duct 10 without having to remove the incontinence device in its entirety.

The device forming the subject-matter of the present invention may be produced from a suitable material, such as silicone rubber, which has a small wall thickness and can be sterilised and cleaned as well as recycled.

## Claims

1. Device for women suffering from incontinence, of the type which comprises a cylindrical sleeve (5) provided, at one end, with a closure head (6) for closing the neck of the bladder, which closure head (6) has transverse openings (7, 8, 9) for communicating with the axial hole (10) of the sleeve (5), and having, at the opposite end, a removable plugging means (11, 12), for the said axial hole (10), and having also a semi-rigid rod (1) provided with a rounded end (2) and with a flange (3) and a holding grip (4) at the other end, for the introduction of the cylindrical sleeve (5) until the neck of the bladder has been closed, **characterized in that** the closure head (6) for closing the neck of the bladder has a rounded frustoconical structure with various openings (7, 8, 9) communicating at different levels the axial hole (10) of the sleeve (5) with the internal cavity of the bladder.

2. Device for women suffering from incontinence according to Claim 1, **characterised in that** the means (11, 12) for plugging the lower end of the sleeve is formed by a projection of a resilient laminar extension (12) of said end of the sleeve, which end is remote from the closure head (6) for the neck of the bladder.

## Patentansprüche

1. Vorrichtung für an Inkontinenz leidende Frauen, welche eine zylindrische Hülle (5) umfaßt, die an einem Ende mit einem Verschlußkopf (6) zum Verschließen des Harnblasenhalses versehen ist, wobei der Verschlußkopf (6) transversale Öffnungen (7, 8, 9) zur Kommunikation mit einem axialen Loch (10) der Hülle (5) aufweist und am gegenüberliegenden Ende Mittel zum lösbaren Verschließen (11, 12) des genannten axialen Lochs (10) trägt und desweiteren einen halbstarren Schaft (1) trägt, der mit einem runden Ende (2) und am anderen Ende mit einem Kragen (3) und einem Rückzugsgriff (4) versehen ist, um die zylindrische Hülle (5) bis zum Erreichen des Verschließens des Harnblasenhalses einzuführen, **dadurch gekennzeichnet, daß** der Verschlußkopf (6) zum Verschließen des Harnblasenhalses eine gerundete kegelstumpfförmige Struktur mit verschiedenen Öffnungen aufweist, die auf unterschiedlichen Niveaus des axialen Lochs (10) der Hülle (5) mit der inneren Harnblasenkavität kommunizieren.

2. Vorrichtung für an Inkontinenz leidende Frauen gemäß Anspruch 1, **dadurch gekennzeichnet, daß** die Mittel (11, 12) zum Verschließen des unteren Endes der Hülle aus einer flachen elastischen Verlängerung (12) des genannten Endes der Hülle besteht, welches Ende von dem Verschlußkopf (6) für den Harnblasenhals entfernt liegt.

## Revendications

1. Dispositif pour femmes souffrant d'incontinence, du type qui comprend une gaine cylindrique (5) munie, sur une extrémité, d'une tête de fermeture (6) destinée à fermer le col de la vessie, laquelle tête de fermeture (6) présente des ouvertures transversales (7,8,9) pour communiquer avec l'orifice axial (10) de la gaine (5) et comportant, sur l'extrémité opposée, des moyens d'obturation amovibles (11,12) pour ledit orifice axial (10) et comportant également une tige semi-rigide (1) munie d'une extrémité arrondie (2) et d'une collerette (3) et d'une poignée de retenue (4) sur l'autre extrémité, pour l'introduction de la gaine cylindrique (5) jusqu'à l'obtention de la fermeture du col de la vessie, **caractérisé en ce que** la tête de fermeture (6) destinée à fermer le col de la vessie présente une structure tronconique arrondie avec des ouvertures diverses communiquant à différents niveaux de l'orifice axial (10) de la gaine (5) avec la cavité interne de la vessie.

2. Dispositif pour femmes souffrant d'incontinence selon la revendication 1, **caractérisé en ce que** les moyens (11,12) destinés à obturer l'extrémité inférieure de la gaine sont constitués par un prolongement laminaire élastique (12) de ladite extrémité de la gaine, laquelle extrémité est éloignée de la tête de fermeture (6) pour le col de la vessie.
